(19) Europäisches Patentamt
European Patent Office
Office européen des brevets

(11) EP 2 155 152 B1

# (12) EUROPEAN PATENT SPECIFICATION

(45) Date of publication and mention of the grant of the patent:
**04.07.2018 Bulletin 2018/27**

(21) Application number: **08750168.0**

(22) Date of filing: **07.05.2008**

(51) Int Cl.:
***A61K 8/49*** *(2006.01)* ***A61Q 5/10*** *(2006.01)*

(86) International application number:
**PCT/EP2008/055654**

(87) International publication number:
**WO 2008/138845 (20.11.2008 Gazette 2008/47)**

(54) **COLOURING COMPOSITION COMPRISING AN AMINOPYRAZOLOPYRIDINE OXIDATION BASE AND EXHIBITING AN ACIDIC PH**

FÄRBUNGSZUSAMMENSETZUNG MIT EINER AMINOPYRAZOLOPYRIDIN-OXIDATIONSBASE UND EINEM SAUREN PH-WERT

COMPOSITION COLORANTE COMPRENANT UNE BASE D’OXYDATION D’AMINOPYRAZOLOPYRIDINE ET PRÉSENTANT UN PH ACIDE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.05.2007 FR 0754952**
**04.06.2007 US 924886 P**

(43) Date of publication of application:
**24.02.2010 Bulletin 2010/08**

(73) Proprietor: **L’Oréal**
**75008 Paris (FR)**

(72) Inventors:
- **SAUNIER, Jean-Baptiste**
**F-75014 Paris (FR)**
- **FADLI, Aziz**
**F-77500 Chelles (FR)**

(74) Representative: **Fevrier, Murielle Françoise E.**
**L’Oréal**
**Service DIPI**
**9 Rue Pierre Dreyfus**
**92110 Clichy (FR)**

(56) References cited:
**EP-A- 1 792 606** **EP-A- 1 792 903**
**FR-A- 2 801 308**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 155 152 B1

## Description

A subject-matter of the invention is a composition for colouring keratinous fibres starting from an acidic composition comprising at least one aminopyrazolopyridine base and a coupler. Another subject-matter of the invention is the use of this composition for the dyeing of keratinous fibres and the dyeing method employing this composition.

It is known to dye keratinous fibres, in particular human hair, with dyeing compositions comprising oxidation dye precursors, generally known as oxidation bases, such as ortho- or para-phenylenediamines, ortho- or para-aminophenols and heterocyclic compounds. These oxidation bases are colourless or weakly coloured compounds which, in combination with oxidizing products, can give rise, by an oxidative coupling process, to coloured compounds.

It is also known that it is possible to vary the shades obtained with these oxidation bases by combining them with couplers or colouring modifiers, the latter being chosen in particular from aromatic meta-diamines, meta-aminophenols, meta-diphenols and certain heterocyclic compounds, such as indole compounds.

The variety of the molecules employed as oxidation bases and couplers makes it possible to obtain a rich palette of colours.

The "permanent" colouring obtained by virtue of these oxidation dyes furthermore has to satisfy a certain number of requirements. Thus, it must be without disadvantage toxicologically, it must make it possible to obtain shades within the desired intensity and it must behave well in the face of external agents, such as light, bad weather, washing, permanent waving, perspiration and rubbing.

The dyes must also make it possible to cover white hair and, finally, be as non-selective as possible, that is to say make it possible to obtain the smallest possible differences in colouring along the same keratinous fibre, which is generally differentially sensitized (i.e. damaged) between its tip and its root.

It is already known to use aminopyrazolopyridine oxidation bases for the dyeing of keratinous fibres, in particular in Patent Application FR 2 801 308. These bases make it possible to obtain varied shades.

The aim of the present invention is to obtain a hair colouring composition which exhibits improved dyeing properties in terms of power and of resistance to external agents.

This aim is achieved with the present invention, a subject-matter of which is a composition for the colouring of keratinous fibres exhibiting a pH range of from 1 to 6.9 and comprising, in an appropriate dyeing medium, one or more couplers and

- one or more aminopyrazolopyridine oxidation bases chosen from the bases of following formula (I):

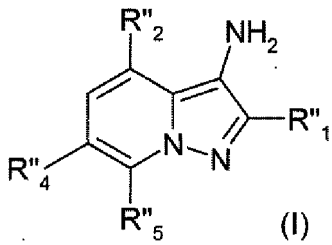

(I)

in which R"$_2$, R"$_4$ and R"$_5$ are chosen independently from a hydrogen atom or a methyl or ethyl group, preferably a hydrogen atom, and R"$_1$ is chosen from:

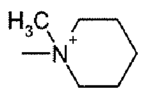

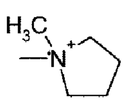

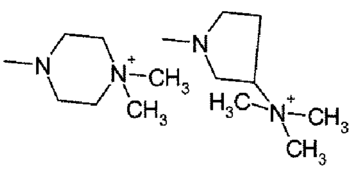

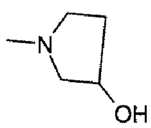

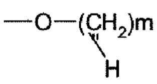

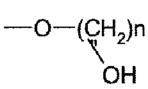

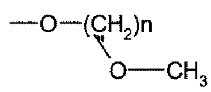

n varying from 2 to 6 and m varying from 0 to 6 in these formulae, and

- one or more oxidizing agents.

Another subject-matter of the invention is a dyeing method employing this composition.

Another subject-matter of the invention is the use of the composition of the present invention for the dyeing of keratinous fibres, in particular human keratinous fibres, such as the hair.

The composition of the present invention makes it possible in particular to obtain a composition for the dyeing of keratinous fibres which is suitable for use in oxidation dyeing and which makes it possible to obtain a colouring with varied, powerful, attractive and not very selective shades which is highly resistant to the various attacks to which hair may be subjected, such as shampoos, light, sweat and permanent deformations.

Mention may be made, as an example of oxidation base of use in the method of the present invention, of:

2-[(3-aminopyrazolo[1,5-a]pyridin-2-yl)oxy]ethanol hydrochloride

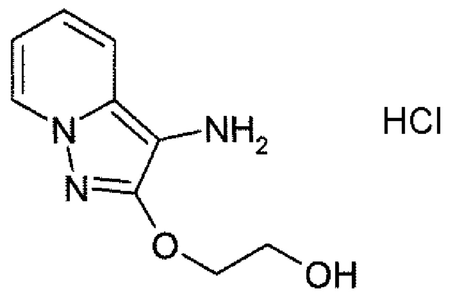

2-methoxy-6,7-dimethylpyrazolo[1,5-a]pyridin-3-amine hydrochloride

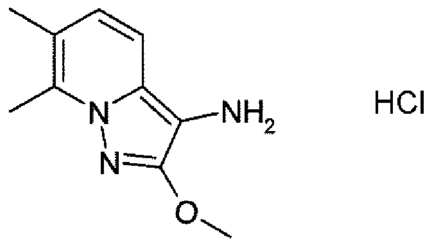

4-ethyl-2-methoxy-7-methylpyrazolo[1,5-a]pyridin-3-amine hydrochloride

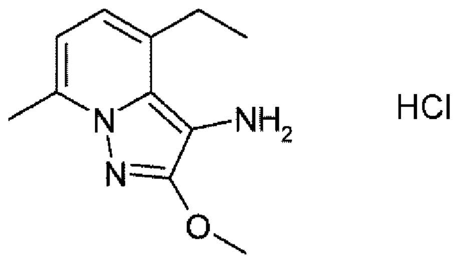

4-(3-aminopyrazolo[1,5-a]pyridin-2-yl)-1,1-dimethylpiperazin-1-ium chloride hydrochloride

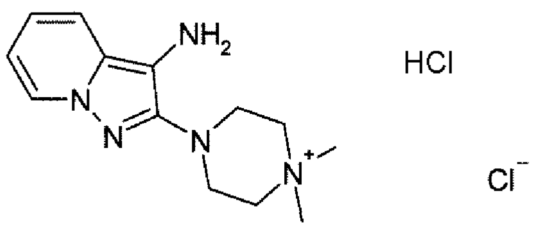

1-(3-aminopyrazolo[1,5-a]pyridin-2-yl)-N,N,N-trimethylpyrrolidin-3-aminium chloride hydrochloride

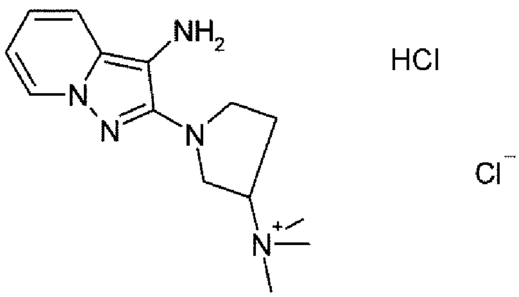

1-(3-aminopyrazolo[1,5-a]pyridin-2-yl)pyrrolidin-3-ol hydrochloride

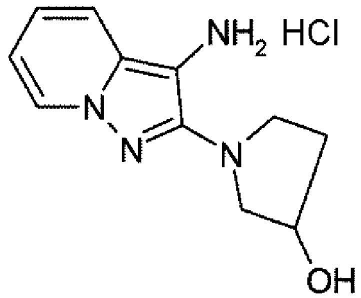

The dyeing composition of the invention comprises one or more couplers conventionally used for the dyeing of keratinous fibres. Mention may in particular be made, among these couplers, of meta-phenylenediamines, meta-aminophenols, meta-diphenols, naphthalene couplers, heterocyclic couplers and their addition salts.

Mention may be made, as example of coupler, of 2-methyl-5-aminophenol, 5-N-(β-hydroxyethyl)amino-2-methylphenol, 6-chloro-2-methyl-5-aminophenol, 3-aminophenol, 2,4-dichloro-3-aminophenol, 5-amino-4-chloro-o-cresol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-(β-hydroxyethylamino)-3,4-methylenedioxybenzene, α-naphthol, 2-methyl-1-naphthol, 1,5-dihydroxynaphthalene, 2,7-naphthalenediol, 1-acetoxy-2-methylnaphthalene, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 2,6-dihydroxy-3-4-dimethylpyridine, 3-amino-2-methylamino-6-methoxypyridine, 1-N-(β-hydroxyethyl)amino-3,4-methylenedioxybenzene, 2,6-bis(β-hydroxyethylamino)toluene, 3-methyl-1-phenyl-5-pyrazolone, and their addition salts with an acid.

According to a specific embodiment, the pH of the composition of use in the method of the present invention is between 3 and 6.8.

The composition of the present invention can also comprise one or more additional oxidation bases conventionally used in oxidation dyeing other than the oxidation bases of formula (I). By way of example, these additional oxidation bases are chosen from para-phenylenediamines other than those described above, bis-phenylalkylenediamines, para-aminophenols, bis-para-aminophenols, ortho-aminophenols, heterocyclic bases other than the oxidation bases of formula (I), and their addition salts.

Mention may be made, among para-phenylenediamines, by way of example, of para-phenylenediamine, para-toluenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-(β-hydroxyethyl)-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl, β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-(β-hydroxyethyloxy)-para-phenylenediamine, 2-(β-acetylaminoethyloxy)-para-phenylenediamine, N-(β-methoxyethyl)-para-phenylenediamine, 4-aminophenylpyrrolidine, 2-thienyl-para-phenylenediamine, 2-(β-hydroxyethylamino)-5-aminotoluene, 3-hydroxy-1-(4'-aminophenyl)pyrrolidine and their addition salts with an acid.

Among the para-phenylenediamines mentioned above, para-phenylenediamine, para-toluenediamine, 2-isopropyl-para-phenylenediamine, 2-(β-hydroxyethyl)-para-phenylenediamine, 2-(β-hydroxyethyloxy)-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine, 2-(β-acetylaminoethyloxy)-para-phenylenediamine and their addition salts with an acid are particularly preferred.

Mention may be made, among bisphenylalkylenediamines, by way of example, of N,N-bis(β-hydroxyethyl)-N,N-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, 1,8-bis(2,5-diaminophenoxy)-3,6-dioxaoctane and their addition salts with an acid.

Mention may be made, among para-aminophenols, by way of example, of para-aminophenol, 4-amino-3-methylphenol, 4-amino-3-fluorophenol, 4-amino-3-(hydroxymethyl)phenol, 4-amino-2-methylphenol, 4-amino-2-(hydroxymethyl)phenol, 4-amino-2-(methoxymethyl)phenol, 4-amino-2-(aminomethyl)phenol, 4-amino-2-((β-hydroxyethyl)aminomethyl)phenol, 4-amino-2-fluorophenol, 1-hydroxy-4-(methylamino)benzene, 2,2'-methylenebis(4-aminophenol) and their addition salts with an acid.

Mention may be made, among ortho-aminophenols, by way of example, of 2-aminophenol, 2-amino-5-methylphenol, 2-amino-6-methylphenol, 5-acetamido-2-aminophenol and their addition salts with an acid.

Mention may be made, among heterocyclic bases, by way of example, of pyridine derivatives, pyrimidine derivatives and pyrazole derivatives.

Mention may be made, among pyridine derivatives, of the compounds described, for example, in Patents GB 1 026 978 and GB 1 153 196, such as 2,5-diaminopyridine, 2-[(4-methoxyphenyl)amino]-3-aminopyridine, 2,3-diamino-6-methoxypyridine, 2-[(β-methoxyethyl)amino]-3-amino-6-methoxypyridine, 3,4-diaminopyridine and their addition salts with an acid.

Mention may be made, among pyrimidine derivatives, of the compounds disclosed, for example, in Patents DE 2 359 399; JP 88-169571; JP 05-63124; EP 0 770 375 or Patent Application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine or 2,5,6-triaminopyrimidine, and pyrazolopyrimidine derivatives, such as those mentioned in Patent Application FR-A-2 750 048 and among which may be mentioned pyrazolo[1,5-a]pyrimidine-3,7-diamine; 2,5-dimethylpyrazolo[1,5-a]pyrimidine-3,7-diamine; pyrazolo[1,5-a]pyrimidine-3,5-diamine; 2,7-dimethylpyrazolo[1,5-a]pyrimidine-3,5-diamine; 3-aminopyrazolo[1,5-a]pyrimidin-7-ol; 3-aminopyrazolo[1,5-a]pyrimidine-5-ol; 2-(3-aminopyrazolo[1,5-a]pyrimidin-7-ylamino)ethanol, 2-(7-aminopyrazolo[1,5-a]pyrimidin-3-ylamino)ethanol, 2-[(3-aminopyrazolo[1,5-a]pyrimidin-7-yl)(2-hydroxyethyl)amino]ethanol, 2-[(7-aminopyrazolo[1,5-a]pyrimidin-3-yl) (2-hydroxyethyl) amino] ethanol, 5,6-dimethylpyrazolo[1,5-a]pyrimidine-3,7-diamine, 2,6-dimethylpyrazolo[1,5-a]pyrimidine-3,7-diamine, 2,5,N7,N7-tetramethylpyrazolo[1,5-a]pyrimidine-3,7-diamine, 3-amino-5-methyl-7-(imidazolylpropylamino)pyrazolo[1,5-a]pyrimidine and their addition salts with an acid and their tautomeric forms, when a tautomeric equilibrium exists.

Mention may be made, among pyrazole derivatives, of the compounds disclosed in Patents DE 3 843 892 and DE 4 133 957 and Patent Applications WO 94/08969, WO 94/08970, FR-A-2 733 749 and DE 195 43 988, such as 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-(tert-butyl)-1-methylpyrazole, 4,5-diamino-1-(tert-butyl)-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-(hydroxymethyl)pyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-(methylamino)pyrazole, 3,5-diamino-4-(β-hydroxyethyl)amino-1-methylpyrazole and their addition salts with an acid.

Mention may also be made of the diaminopyrazolinones described in Patent Application FR 2 886 137 and in particular 2,3-diamino-6,7-dihydro-1H,5H-pyrazol-1-one and its salts.

Generally, the addition salts of the oxidation bases and couplers which can be used in the context of the invention are chosen in particular from the addition salts with an acid, such as hydrochlorides, hydrobromides, sulphates, citrates, succinates, tartrates, lactates, tosylates, benzenesulphonates, phosphates and acetates, and the addition salts with a base, such as sodium hydroxide, potassium hydroxide, aqueous ammonia, amines or alkanolamines.

The dyeing composition in accordance with the invention can additionally comprise one or more direct dyes which can in particular be chosen from nitrobenzene dyes, azo direct dyes or methine direct dyes. These direct dyes can be nonionic, anionic or cationic in nature.

The medium appropriate for dyeing, also referred to as dyeing vehicle, generally comprises water or a mixture of water and at least one organic solvent in order to dissolve the compounds which would not be sufficiently soluble in water. Mention may be made, as an organic solvent, for example, of lower $C_1$-$C_4$ alkanols, such as ethanol and isopropanol; polyols and polyol ethers, such as 2-butoxyethanol, propylene glycol, propylene glycol monomethyl ether, diethylene glycol monoethyl ether and diethylene glycol monomethyl ether; and also aromatic alcohols, such as benzyl alcohol or phenoxyethanol, and their mixtures.

The solvents are preferably present in proportions preferably of between 1 and 40% by weight approximately, with respect to the total weight of the dyeing composition, and more preferably still between 5 and 30% by weight approximately.

The dyeing composition in accordance with the invention can also include various adjuvants conventionally used in compositions for dyeing the hair, such as anionic, cationic, nonionic, amphoteric or zwitterionic surface-active agents or their mixtures, anionic, cationic, nonionic, amphoteric or zwitterionic polymers or their mixtures, inorganic or organic thickening agents and in particular anionic, cationic, nonionic and amphoteric polymeric associative thickeners, antioxidants, penetration agents, sequestering agents, fragrances, buffers, dispersing agents, conditioning agents, such as, for example, volatile or non-volatile and modified or unmodified silicones, film-forming agents, ceramides, preservatives or opacifying agents.

The above adjuvants are generally present in an amount, for each of them, of between 0.01 and 20% by weight, with respect to the weight of the composition.

Of course, a person skilled in the art will take care to choose this or these optional additional compounds so that the advantageous properties intrinsically attached to the oxidation dyeing composition in accordance with the invention are not, or not substantially, detrimentally affected by the envisaged addition or additions.

The pH of the dyeing composition in accordance with the invention ranges from 1 to 6.9 and preferably from 4 to 6.9 approximately. It can be adjusted to the desired value using acidifying and/or basifying agents commonly used in the dyeing of keratinous fibres or else by means of conventional buffering systems.

Mention may be made, among acidifying agents, by way of example, of inorganic or organic acids, such as hydrochloric acid, orthophosphoric acid, sulphuric acid, carboxylic acids, such as acetic acid, tartaric acid, citric acid or lactic acid or sulphonic acids.

Mention may be made, among basifying agents, by way of example, of aqueous ammonia, alkaline carbonates, alkanolamines, such as mono-, di- and triethanolamines and their derivatives, sodium hydroxide, potassium hydroxide and the compounds of following formula (II):

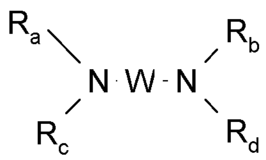

(II)

in which W is a propylene residue optionally substituted by a hydroxyl group or a $C_1$-$C_4$ alkyl radical and $R_a$, $R_b$, $R_c$ and $R_d$, which are identical or different, represent a hydrogen atom or a $C_1$-$C_4$ alkyl or $C_1$-$C_4$ hydroxyalkyl radical.

The oxidizing agent or agents present in the composition of the invention are, for example, hydrogen peroxide, urea hydrogen peroxide, alkali metal bromates, persalts, such as perborates and persulphates, or peracids. Hydrogen peroxide is particularly preferred.

The dyeing composition according to the invention can be provided in various forms, such as in the form of liquids, of creams or of gels or in any other form appropriate for carrying out dyeing of keratinous fibres and in particular of human hair.

The method of the present invention is a method in which the composition according to the present invention as defined above is applied to the fibres for a time sufficient to develop the desired colouring. The oxidizing agent or agents of the composition according to the invention can be added only at the time of use.

The composition according to the invention is applied to the keratinous fibres. After a setting time of 3 to 50 minutes approximately, preferably 5 to 30 minutes approximately, the keratinous fibres are rinsed, washed with shampoo, rinsed again and then dried.

Another subject-matter of the invention is a dyeing kit or multicompartment device in which a first compartment includes a composition comprising one or more oxidation bases of formula (I) and a second compartment includes an oxidizing agent, the mixture of the contents of the two compartments being in accordance with the invention, exhibiting a pH ranging from 1 to 6.9. This device can be equipped with a means allowing the desired mixture to be deposited on the hair, such as the devices described in Patent FR-2 586 913 on behalf of the Applicant Company.

Starting from this device, it is possible to dye keratinous fibres starting from a method which comprises the mixing of a dyeing composition comprising at least one oxidation base of formula (I) with an oxidizing agent and the application of the mixture obtained to the keratinous fibres for a time sufficient to develop the desired colouring.

The examples which follow serve to illustrate the invention without, however, exhibiting a limiting nature.

## EXAMPLES

### DYEING EXAMPLES

Compositions 1 to 4 comprise the following dyes:

| In moles per 100 g of composition | B1 | B2 | C1 | C2 | C3 | C4 |
|---|---|---|---|---|---|---|
| Composition 1 | 0.002 | | 0.002 | | | |
| Composition 2 | 0.0025 | | | 0.0025 | | |
| Composition 3 | 0.003 | | | | 0.003 | |
| Composition 4 | | 0.004 | | | | 0.004 |
| B1= 2-[(3-Aminopyrazolo[1,5-a]pyridin-2-yl)amino]-N,N,N-trimethylethanaminium chloride hydrochloride<br>B2= 2-[(3-Aminopyrazolo[1,5-a]pyrdin-2-yl)oxy]ethanol hydrochloride<br>C1= 2,4-Diaminophenoxyethanol<br>C2= meta-Aminophenol<br>C3= 2-Chloro-6-methyl-3-aminophenol<br>C4= 1-Methyl-2-hydroxy-4-aminobenzene | | | | | | |

These colouring compositions were introduced into the following vehicle:

| | |
|---|---|
| Polyglycerolated oleyl alcohol comprising 2 mol of glycerol | 4 g A.M. |
| Polyglycerolated oleyl alcohol comprising 4 mol of glycerol (78% A.M.) | 6 g A.M. |
| Oleic acid | 3 g |
| Oleylamine 2 EO, sold under the name of Ethomeen 0/12 by Akzo | 7 g A.M. |
| Diethylaminopropyl laurylaminosuccinamate, sodium salt, comprising 55% A.M. | 3 g A.M. |
| Oleyl alcohol | 5 g |
| Alkyl ($C_{13}$/$C_{15}$ 70/30 50% linear) ether carboxylic acid monoethanolamide (2 EO) | 10 g A.M. |
| Propylene glycol | 12 g |
| Ethyl alcohol | 5 g |
| Sodium metabisulphite as an aqueous solution comprising 35% A.M. | 0.455 g A.M. |
| Ammonium acetate | 0.8 g |
| Antioxidant, sequestering agent | q.s. |
| Fragrance, preservative | q.s. |
| Demineralized water | q.s. for 100 g |

### Method of application

The composition is diluted at the time of use with 1 times its weight of 20-volume aqueous hydrogen peroxide solution.

The pH values of the mixtures are:

| | pH values of the mixtures with aqueous hydrogen peroxide solution |
|---|---|
| Composition 1 | 5.5 |
| Composition 2 | 6.8 |
| Composition 3 | 6.8 |
| Composition 4 | 6.5 |

The mixture thus produced is applied to natural grey hair comprising 90% of white hairs (NW) in a proportion of 30 g per 3 g of hair for 30 min.

The hair is subsequently rinsed, washed with a standard shampoo and dried.

**Evaluation**

The hair colouring is evaluated visually.

| | Height of tone | Highlight |
|---|---|---|
| Composition 1 | Light blond | Blue green |
| Composition 2 | Light blond | Grey |
| Composition 3 | Light blond | Purplish blue |
| Composition 4 | Light blond | Red |

These shades are long-lasting and uniform.

Example 2

composition 5 were prepared :

| | |
|---|---|
| Pyrazolo[1,5-a]pyridin-3amine (0.003 mol) | 0.51 g |
| 2,4-Diaminophenoxyethanol, HCl (0.003)hydrochloride g | 0.723 |
| Ethanol at 96° | 18 g |
| buffer $K_2HPO_4$ / $KH_2PO_4$ (1,5 M / 1M) | 10 g |
| sodium Metabisulphite | 0.68 g |
| diethylenetriaminopentacetic acid pentasodic salt | 1.1 g |
| water Q.S.P | 100 g |

Composition 6 was prepared :

| | |
|---|---|
| 2-[(3-Aminopyrazolo[1,5-a]pyridin-2-yl)oxy]ethanol hydrochloride (0.003 mol) | 0.69 g |
| 2,4-Diaminophenoxyethanol, HCl (0.003) | 0.723 g |
| Ethanol at 96° | 18 g |
| buffer $K_2HPO_4$ / $KH_2PO_4$ (1,5 M / 1M) | 10 g |
| sodium Metabisulphite | 0.68 g |
| diethylenetriaminopentacetic acid pentasodic salt | 1.1 g |
| water Q.S.P | 100 g |

**Method of application**

The compositions were mixed at the time of use with 1 time its weight of 20-volum hydrogen peroxidewith a pH of 3.

After mixing, pH of the compositions 5 and 6 were 5.7± 0.1.

The resulting mixtures were applied to natural or bleached grey hair comprising 90% of white hairs in a proportion of 20 g per 2 g of hair for 30 min.

The hair is subsequently rinsed, washed with a standard shampoo and dried.

**Color determination**

The color of the hair was evaluated in the L*a*b* system, with a spectrophotometer SF600X de Datacolor (diffus, 8°) : opening SAV, mesure in SCI, UVI with illuminant D65.

According to this system, L* indicates the lightness. The lowest is the value of L, the most intense is the color of the hair. The chromaticity coordinates are expressed by the parameters a* and b*, a* indicating the axis of red/green shades and b* the axis of yellow/blue shades.

## SELECTIVITY OF THE COLORATION

The selectivity of the coloration is the variation of the color between natural colored hair and bleached colored hair. Natural hair is representative of the nature of the hair at the point hair and the bleached hair is representative of the nature of the hair at the root.

The selectivity is measured by :

ΔE, which is the color variation between a natural colored lock and a bleached colored lock, is obtained from the following formula:

$$\Delta E = \sqrt{(L^* - L_o{}^*)^2 + (a^* - a_o{}^*)^2 + (b^* - b_o{}^*)^2}$$

wherein L* indicates lightness and a* and b* are the chromaticity coordinates of the natural colored locks whereas $L_0$* indicates the lightness and $a_C$* et $b_C$* are the chromaticity of the bleached colored locks. The lowest is the value of ΔE, the weakest the selective is the coloration and the best is the color of the hair.

The results are reported in the following table.

| | *After coloration of natural hair* | | | *After coloration of bleached hair* | | | *Selectivity* |
|---|---|---|---|---|---|---|---|
| | *L** | *a** | *b** | *L** | *a** | *b** | |
| Composition 5 | 24.1 | 17.1 | 1.0 | 18.2 | 4.8 | 0.9 | **13.6** |
| Composition 6 | 21.7 | 10.6 | 3.1 | 18.5 | 3.0 | 1.3 | **8.4** |

These results show a lower selectivity for composition 6 than the one obtained from comparative composition 5.

## Claims

1. Composition for the colouring of keratinous fibres exhibiting a pH range of from 1 to 6.9 and comprising, in an appropriate dyeing medium, one or more couplers and

   • one or more aminopyrazolopyridine oxidation bases chosen from the bases of following formula (I):

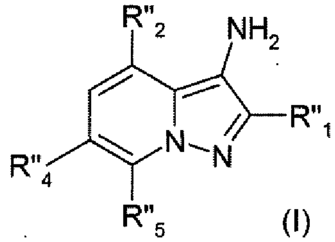

(I)

   in which R"$_2$, R"$_4$ and R"$_5$ are chosen independently from a hydrogen atom or a methyl or ethyl group, and R"$_1$ is chosen from:

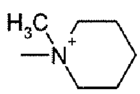

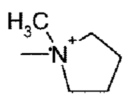

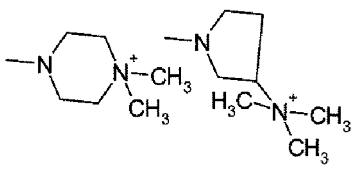

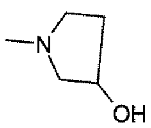

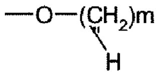 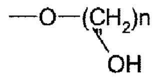 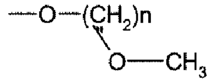

n varying from 2 to 6 and m varying from 0 to 6 in these formulae, and
• one or more oxidizing agents.

**2.** Composition according to Claim 1, in which the R"$_2$, R"$_4$ and R"$_5$ radicals are hydrogen atoms.

**3.** Composition according to either one of the preceding claims, in which the pH of the composition ranges from 4 to 6.9.

**4.** Composition according to Claim 1 or 2, in which the oxidation base or bases are chosen from:

2-[(3-aminopyrazolo[1,5-a]pyridin-2-yl)oxy]ethanol hydrochloride

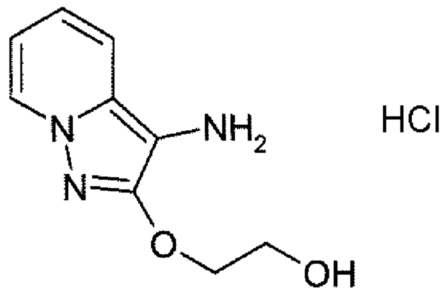

2-methoxy-6,7-dimethylpyrazolo[1,5-a]pyridin-3-amine hydrochloride

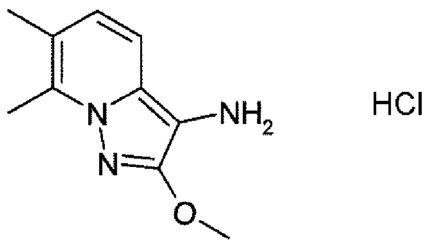

4-ethyl-2-methoxy-7-methylpyrazolo[1,5-a]pyridin-3-amine hydrochloride

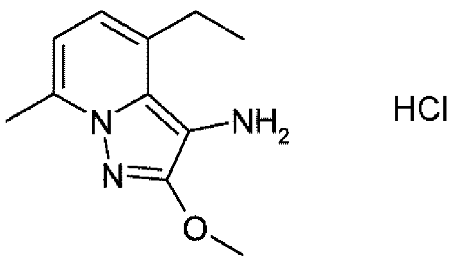

4-(3-aminopyrazolo[1,5-a]pyridin-2-yl)-1,1-dimethyl-piperazin-1-ium chloride hydrochloride

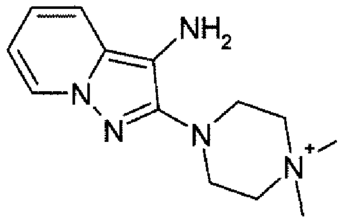

$HCl$

$Cl^-$

5. Composition according to any one of the preceding claims, additionally comprising one or more additional oxidation bases chosen from para-phenylenediamines, bisphenylalkylenediamines, para-aminophenols, ortho-aminophenols, heterocyclic bases other than the oxidation bases of formula (I), and their addition salts.

6. Composition according to any one of the preceding claims, in which the coupler or couplers are chosen from meta-phenylenediamines, meta-aminophenols, meta-diphenols, naphthalene couplers, heterocyclic couplers and their addition salts.

7. Composition according to any one of the preceding claims, comprising an amount of each of the oxidation bases of between 0.001 and 10% by weight approximately of the total weight of the dyeing composition.

8. Composition according to any one of the preceding claims, comprising an amount of each of the couplers of between 0.001 and 10% by weight approximately of the total weight of the dyeing composition.

9. Composition according to any one of the preceding claims, in which the oxidizing agent or agents are chosen from hydrogen peroxide, urea hydrogen peroxide, alkali metal bromates, persalts, such as perborates and persulphates, or peracids.

10. Composition according to the preceding claim, in which the oxidizing agent is hydrogen peroxide.

11. Method for colouring keratinous fibres, **characterized in that** a dyeing composition as defined in any one of the preceding claims is applied to the fibres for a time sufficient to develop the desired colouring.

## Patentansprüche

1. Zusammensetzung zum Färben von Keratinfasern, die einen pH-Bereich von 1 bis 6,9 aufweist und in einem geeigneten Färbemedium ein oder mehrere Kuppler und

    • eine oder mehrere Aminopyrazolopyridin-Oxidationsbasen, die aus den Basen der folgenden Formel (I) ausgewählt sind:

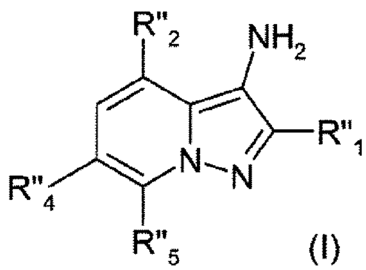

(I)

wobei R"$_2$, R"$_4$ und R"$_5$ unabhängig aus einem Wasserstoffatom oder einer Methyl- oder Ethylgruppe ausgewählt sind und R"$_1$ aus:

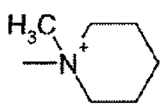

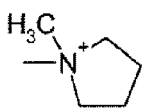

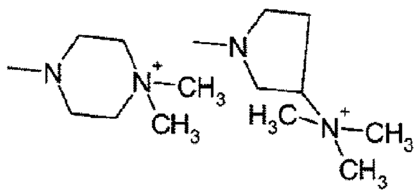

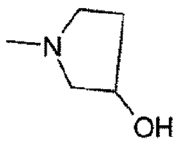

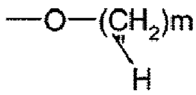

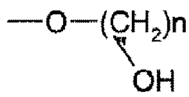

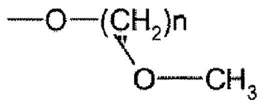

wobei in diesen Formeln n von 2 bis 6 variiert und m von 0 bis 6 variiert, ausgewählt ist, und

• ein oder mehrere Oxidationsmittel umfasst.

**2.** Zusammensetzung nach Anspruch 1, wobei die R"$_2$-, R"$_4$- und R''$_5$-Reste für Wasserstoffatome stehen.

**3.** Zusammensetzung nach einem der vorhergehenden Ansprüche, wobei der pH-Wert der Zusammensetzung im Bereich von 4 bis 6,9 liegt.

**4.** Zusammensetzung nach Anspruch 1 oder 2, wobei die Oxidationsbase bzw. Oxidationsbasen aus 2-[(3-Aminopyrazolo[1,5-a]pyridin-2-yl)oxy]-ethanol-hydrochlorid

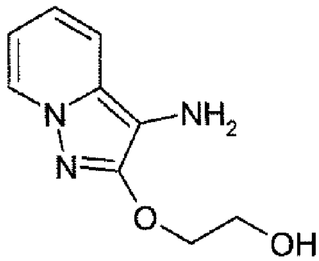

HCl

2-Methoxy-6,7-dimethylpyrazolo[1,5-a]pyridin-3-amin-hydrochlorid

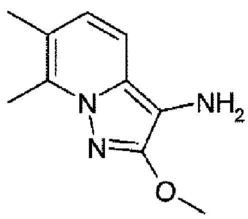

HCl

4-Ethyl-2-methoxy-7-methylpyrazolo[1,5-a]pyridin-3-amin-hydrochlorid

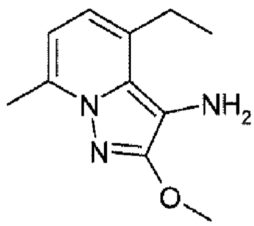

HCl

4-(3-Aminopyrazolo[1,5-a]pyridin-2-yl)-1,1-dimethylpiperazin-1-iumchlorid-hydrochlorid

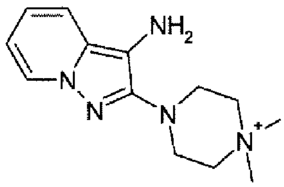

HCl

$Cl^-$

ausgewählt ist bzw. sind.

**5.** Zusammensetzung nach einem der vorhergehenden Ansprüche, die zusätzlich eine oder mehrere zusätzliche Oxidationsbasen, die aus paraPhenylendiaminen, Bisphenylalkylendiaminen, para-Aminophenolen, ortho-Aminophenolen, heterocyclischen Basen, die von den Oxidationsbasen der Formel (I) verschieden sind, und Additionssalzen davon ausgewählt sind, umfasst.

**6.** Zusammensetzung nach einem der vorhergehenden Ansprüche, wobei der Kuppler bzw. die Kuppler aus meta-Phenylendiaminen, meta-Aminophenolen, meta-Diphenolen, Naphthalin-Kupplern, heterocyclischen Kupplern und Additionssalzen davon ausgewählt sind.

**7.** Zusammensetzung nach einem der vorhergehenden Ansprüche, die eine Menge jeder der Oxidationsbasen zwischen etwa 0,001 und 10 Gew.-%, bezogen auf das Gesamtgewicht der Färbezusammensetzung, umfasst.

**8.** Zusammensetzung nach einem der vorhergehenden Ansprüche, die eine Menge jedes der Kuppler zwischen etwa 0,001 und 10 Gew.-%, bezogen auf das Gesamtgewicht der Färbezusammensetzung, umfasst.

**9.** Zusammensetzung nach einem der vorhergehenden Ansprüche, wobei das Oxidationsmittel bzw. die Oxidationsmittel aus Wasserstoffperoxid, Harnstoff-Wasserstoffperoxid, Alkalimetallbromaten, Persalzen, wie Perboraten und Persulfaten, oder Persäuren ausgewählt ist bzw. sind.

**10.** Zusammensetzung nach dem vorhergehenden Anspruch, wobei es sich bei dem Oxidationsmittel um Wasserstoffperoxid handelt.

**11.** Verfahren zum Färben von Keratinfasern, **dadurch gekennzeichnet, dass** man auf die Fasern eine Färbezusammensetzung gemäß einem der vorhergehenden Ansprüche über einen zur Entwicklung der gewünschten Färbung ausreichenden Zeitraum aufbringt.

**Revendications**

**1.** Composition de coloration de fibres kératiniques présentant un pH allant de 1 à 6, 9 et comprenant, dans un milieu de teinture approprié, un ou plusieurs coupleurs et

• une ou plusieurs bases d'oxydation aminopyrazolopyridine choisies parmi les bases de formule (I) suivante :

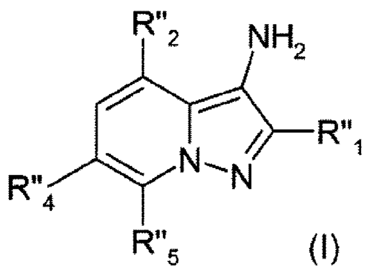

(I)

dans laquelle $R''_2$, $R''_4$ et $R''_5$ sont indépendamment choisis parmi un atome d'hydrogène, un groupe méthyle ou éthyle, et $R''_1$ est choisi parmi :

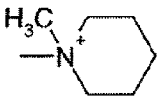
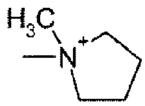

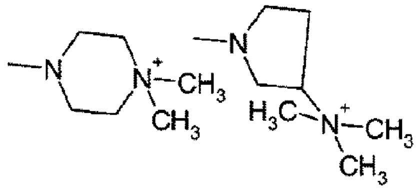
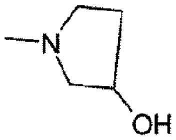

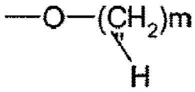
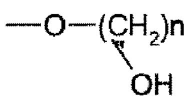
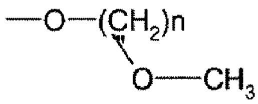

n varient de 2 à 6 et m variant de 0 à 6 dans ces formules, et

- un ou plusieurs agents oxydants.

**2.** Composition selon la revendication 1, dans laquelle les radicaux $R''_2$, $R''_4$ et $R''_5$ sont des atomes d'hydrogène.

**3.** Composition selon l'une quelconque des revendications précédentes, dans laquelle le pH de la composition va de 4 à 6,9.

**4.** Composition selon la revendication 1 ou 2, dans laquelle la ou les bases d'oxydation sont choisies parmi :

2[(3-aminopyrazolo[1,5-a]pyridin-2-yl)oxy]éthanol hydrochlorure

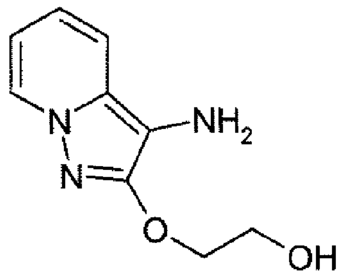
HCl

2-méthoxy-6,7-diméthylpyrazolo[1,5-a]pyridin-3-amine hydrochlorure

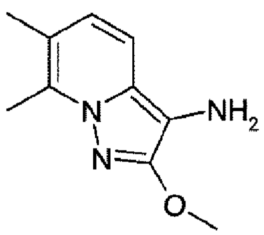
HCl

4-éthyl-2-méthoxy-7-méthylpyrazolo[1,5-a]pyridin-3-amine hydrochlorure

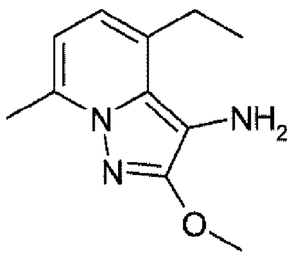

HCl

4-(3-aminopyrazolo[1,5-a]pyridin-2-yl)-1,1-diméthylpipérazin-1-ium chlorure hydrochlorure

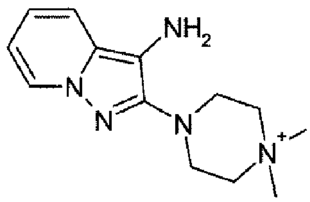

HCl

$Cl^-$

.

5. Composition selon l'une quelconque des revendications précédentes, comprenant de plus une ou plusieurs base d'oxydation additionnelles choisies parmi les para-phénylènediamines, les bis-phénylalkylènediamines, les para-aminophénols, les orthoaminophénols, les bases hétérocycliques autres que les bases d'oxydation de formule (I) et leurs sels d'addition.

6. Composition selon l'une quelconque des revendications précédentes, dans laquelle le ou les coupleurs sont choisis parmi les métaphénylènediamines, les métaaminophénols, les métadiphénols, les coupleurs naphtaléniques, les coupleurs hétérocycliques et leurs sels d'addition.

7. Composition selon l'une quelconque des revendications précédentes, comprenant une quantité de chacune des bases d'oxydation comprise entre 0,001 et 10 % en poids environ du poids total de la composition tinctoriale.

8. Composition selon l'une quelconque des revendications précédentes, comprenant une quantité de chacun des coupleurs comprise entre 0,001 et 10 % en poids environ du poids total de la composition tinctoriale.

9. Composition selon l'une quelconque des revendications précédentes, dans laquelle le ou les agents d'oxydation sont choisis parmi le peroxyde d'hydrogène, le peroxyde d'hydrogène d'urée, les bromates de métaux alcalins, les persels tels que les perborates et persulfates, les peracides.

10. Composition selon la revendication précédente, dans laquelle l'agent d'oxydation est le peroxyde d'hydrogène.

11. Procédé de coloration de fibres kératiniques, **caractérisé en ce qu'**une composition tinctoriale telle que définie dans l'une quelconque des revendications précédentes est appliquée sur les fibres pendant un temps suffisant pour développer la coloration désirée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2801308
- GB 1026978 A
- GB 1153196 A
- DE 2359399
- JP 63169571 A
- JP 5063124 A
- EP 0770375 A
- WO 9615765 A
- FR 2750048 A
- DE 3843892
- DE 4133957
- WO 9408969 A
- WO 9408970 A
- FR 2733749 A
- DE 19543988
- FR 2886137
- FR 2586913